# EUROPEAN PATENT SPECIFICATION

(11) **EP 3 348 540 B1**
(45) Date of publication and mention of the grant of the patent: **17.02.2021**
(21) Application number: 15903694.6
(22) Date of filing: 25.12.2015
(51) Int. Cl.: C05F 11/08, C12R 1/01, A01N 63/00

(54) **BEIJERINCKIA FLUMINENSIS BF 2806 BACTERIAL STRAIN, USE THEREOF AS A FERTILIZER AND A BIOLOGICAL CONTROL AGENT IN PREVENTING AND/OR TREATING PLANT DISEASES, AND METHOD OF STIMULATING PLANT GROWTH AND PROTECTING PLANTS AGAINST DISEASES**
BEIJERINCKIA-FLUMINENSIS-BF-2806-BAKTERIENSTAMM, SEINE VERWENDUNG ALS DÜNGEMITTEL UND BIOLOGISCHES SCHÄDLINGSBEKÄMPFUNGSMITTEL ZUR VORBEUGUNG UND/ODER BEHANDLUNG VON PFLANZENBEFALL UND VERFAHREN ZUR STIMULIERUNG DES PFLANZENWACHSTUMS UND ZUM SCHUTZ VON PFLANZEN VOR KRANKHEITEN
SOUCHE BACTÉRIENNE BEIJERINCKIA FLUMINENSIS BF 2806, SON UTILISATION EN TANT QUE ENGRAIS ET AGENT DE CONTRÔLE BIOLOGIQUE DANS LA PRÉVENTION ET/OU LE TRAITEMENT DES MALADIES DE VÉGÉTAUX ET PROCÉDÉ DE STIMULATION DE CROISSANCE ET DE PROTECTION DES VÉGÉTAUX CONTRE LES MALADIES

(30) Priority: 10.09.2015 RU 2015138550
(43) Date of publication of application: 18.07.2018
(73) Proprietor: OOO "Promyshlennye Innovatsii", Moscow 127486 (RU)
(72) Inventor: PLASTININ, Sergey Arkadievich, Arhangelievskaya obl. 164070 (RU); ZDORNOV, Aleksey Vyacheslavovich, Zelenograd Moscow 124482 (RU); NIKULSHIN, Vadim Albertovich, Chuvashskaya Respublika 428010 (RU)
(74) Representative: Boskovic, Davor
(86) International application number: PCT/RU2015/000930
(87) International publication number: WO 2017/043998

(56) References cited:
- WO-A1-87/03303
- BR-A2- PI0 804 529
- RU-C1- 2 148 571
- US-B1- 6 939 688

## Description

### FIELD OF THE INVENTION

The invention related to agricultural microbiology, in particular to Beijerinckia fluminensis strain Bf 2806 (registration number VKPM B-12258), used as a fertilizer, biological control agent in the prevention and/or treatment of fungal diseases in agricultural and ornamental plants and a method for plant growth stimulation and protection of agricultural and ornamental plants against fungal diseases.

### BACKGROUND OF THE INVENTION

Currently, the use of bacterial products to improve soil fertility is one of the agrotechnology techniques, an alternative to the increasing use of mineral fertilizers. This is especially relevant due to the required environmental safety.

Bioadditives accelerate the release of nutrients from organic fertilizers, as well as the process of assimilation of the released substances by the plants. In addition, bioadditives contribute to a further accumulation of nutrients in the soil in the form easily digestible by plants.

The use of bacterial fertilizers on the basis of various strains of microorganisms or their metabolites is known.

Thus, the inclusion of an aqueous suspension of bacterial cultures (bacterial stock culture of AMB and azobacterin) into the mixture of peat, lime and mineral additives increases the effectiveness of the fertilizer (SU 589238).

The inclusion of a suspension of yeast and unicellular algae (SU 935501), consortium of bacteria Streptococcus thermophilus, Streptococcus bovis, Dactobacillus salivaries var salicinicus, Lactobacillus salivaries var salicinicus, Lactobacillus acidophilus (RU 2055823), a suspension of nitrogen-fixing (Azotobacter chroococcum), phosphate-solubilizing (Bacillus mucilaginosus), lactic acid (as a consortium containing Streptococcus thermophilus, Streptococcus bovis, Lactobacillus salivaries var salicinicus, Lactobacillus salivaries var salivarus, Lactobacillus acidophilus (RU 2081866) bacteria into organomineral fertilizers to increase their effectiveness is known.
Fertilizers are known for the preparation of which strains cultivated in nutrient media containing peptone (RU 2241692) or nitrogen compounds (SU 1756318) are used, which boosts the vegetative mass of plants in respect to fertilizers based on the strain and Agrobacterium radiobacter, respectively. The application of a biological agent aimed at increasing the yield of agricultural crops and improving the product quality is described, containing strains of such genera as Agrobacterium, Bacillus, Bradyrhizobium, Ervinia, Rhodopseudomonas (RU 2322061) in addition to lactic acid, photosynthetic and nitrogen-containing bacteria.

The prior art describes a fertilizer based on the strain of bacteria Bacillus mucilaginosus - Bac 1208 able to produce exopolysaccharide which creates a beneficial microflora for the rhizosphere (RU2081867).
The application of a bioadditive acting as a fertilizer (RU 2148571) is described, which consists of associations of nitrogen-fixing bacteria Azotobacter chroococcum GSB-TB 4B and Beijerinckia fluminensis GSB-TB 5B taken in a ratio of (1 - 0.5): (1 - 0.5), as well as bacteria Bacillus megaterium GSB-TB 3B and Bacillus mucilaginosus GSB-TB 6B breaking down phosphorus- and potassium-containing compounds, taken in the ratio (1 - 5 : 0.5 - 2).

The strain of bacteria Azotobacter chroococcum BH-1811 VKPM B-9029 used to produce a biological agent intended to increase the yield of crops and improve their resistance to various diseases is described in patent RU 2289620.
Patent RU 2408721 describes the strain of bacteria Agrobacterium radiobacter Ag 1108 (VKPM B-10210), having increased nitrogen-fixing properties, as well as a fertilizer on its basis.

A strain of microorganisms Sphingobacterium multivorum is proposed for use in the microbiological industry for obtaining bacterial fertilizers for tomatoes and cucumbers (RF patent 2458119).

The application of a strain of Pseudomonas brassicacearum for the production of a fertilizer is described (RF patent 2453596).

The use of strains of microorganisms, such as Serratia plymuthica (WO/2012.095431), the fungus of the genus Trichoderma (RF patent 2534213), or a composition of Pseudomonas strains mixed with a humate fertilizer and broth culture of Chlorella Vulgaris (RF patent 2291620) for the biological control of plant pathogens is known. WO 87/03303 A1 discloses agricultural-chemical-producing endoszmbiotic microorganisms and method of preparing comprising the use of nitrogen fixing bacteria such as Beijerinckia.RU 2 148 571 C1 and US 6,939,688 B1 disclose biological additives comprising two bacteria association, namely Azotobacter chroococcum and Beijerinckia fluminensis.

However, known bioadditives used as fertilizers or for protecting plants against phytopathogens have a number of disadvantages due to poor effectiveness and complexity of synthesizing the fertilizer and a narrow spectrum of suppression of phytopathogenic microflora. Moreover, most of the known bioadditives used as fertilizers do not show any antagonistic activity towards phytopathogens.

Thus, the isolation and development of new strains of microorganisms that enhance the effectiveness of organomineral fertilizers and the yield of cultivated plants, with antagonistic activity towards phytopathogens is an important task, the solution of which opens the possibility in principle to achieve on an industrial scale high yields of agricultural crops and high quality of the agricultural products while reducing the amount of fertilizers applied to the soil, as well as to reduce the amount of or eliminate the use of chemicals to protect plants from pathogenic microorganisms.

### Summary of the Invention

The authors of this invention have proposed for the first time the strain of bacteria Beijerinckia fluminensis bacteria Bf 2806 (deposited in the All-Russian National Collection of Industrial Microorganisms (VKPM) under the number VKPM-12258), which enhances the formation of metabolic products accelerating the growth of the vegetative system of plants and stimulating the production of phytohormones by plants.

Beijerinckia fluminensis strain Bf 2806 has an enhanced ability to synthesize bacteriocins with a wide spectrum of phytopathogenic microflora suppression and contributes to an increased yield of a wide range of agricultural crops and ornamental plants.

In one aspect, the invention is focused on Beijerinckia fluminensis strain Bf 2806 VKPM-12258.

In another aspect, the invention relates to a method for stimulating plant growth and protection against fungal diseases involving the use of Beijerinckia fluminensis Bf 2806 VKPM-12258

One more aspect of the invention relates to the use of the Beijerinckia fluminensis strain Bf 2806 VKPM-12258 as a fertilizer and a biological control agent in the prevention or treatment of fungal diseases in plants.

### Detailed Description of the Invention

This invention is based on the surprising discovery that Beijerinckia fluminensis strain Bf 2806 VKPM -12258 can be used as a highly effective fertilizer stimulating the growth of the vegetative mass and increasing the yield of agricultural crops and ornamental plants, and also as a control agent in the prevention and treatment of plant diseases.

The known Beijerinckia fluminensis strain GSB-TB 5B is used as a bioadditive to organomineral fertilizers in a composition with Azotobacter chroococcum, Bacillus megaterium and Bacillus mucilaginosus, taken at a certain ratio, and does not possess any anti-phytopathogenic properties.

In accordance with the invention, Beijerinckia fluminensis strain Bf 2806 VKPM B-12258 was isolated from cultivated soils by way of a multistage selection method and cultivated on a Fedorov's medium (Example 1).

The morphological and cultural charecteristics of Beijerinckia fluminensis strain Bf 2806 VKPM B-12258:
When grown on Fedorov's agar nutrient medium, at a temperature of 28 ± 5 °C for 90-100 hours, the culture forms round convex shiny mucous colonies of 2-4 mm, smooth with an even edge, opaque, of viscous consistency. Under the microscope on hours 18-20 of growth, small mobile rods of 1.0 × 2.0 µm are seen, the length varies up to the coccoid form, in a mature culture (on hours 48-50 of growth), truncated cells are mostly singular, immobile.

### Physiological and biochemical characteristics:

Beijerinckia fluminensis Bf 2806 (VKPM B-12258) is an aerobe, its optimum growth temperature is 30 ± 5 °C, sucrose, glucose, mannose are utilized as a carbon source. The strain uses ammonium, nitrite and nitrate nitrogen, as well as free (atmospheric) nitrogen for its development. Beijerinckia fluminensis strain Bf 2806 VKPM B-12258 is grown in a fermenter to a concentration of 5-30 billion cells per ml of solution at pH of 6.0-7.5 (Example 1).

In one of variants of the invention, the fermentation product, which is a suspension of active bacterial cells of Beijerinckia fluminensis strain Bf 2806 VKPM B-12258 with a titer of at least 1-5 billion cells/ml, is used as a fertilizer.

According to the invention, Beijerinckia fluminensis strain Bf 2806 is used as a fertilizer for cereals (wheat, barley, oats, rye, rice), potatoes, vegetables, berries, flowers and ornamental plants, legumes, coniferous, deciduous, ornamental trees and shrubs. The effectiveness of the claimed strain when used as a fertilizer has been studied in the field on various plants.

When the fertilizer on the basis of the claimed strain is used, the development of bacterial colonies leads to an activation of useful agroflora; due to the activity of the microorganisms, the content of atmospheric nitrogen in the soil increases, the phytohormonal state of the rhizosphere is improved and, as a consequence, the vegetative mass of the plant grows intensively.

In field tests of the Beijerinckia fluminensis strain Bf. 2806 biomass on winter wheat, both with presowing seed treatment and crop treatment at an early stage of the vegetative period, a significant increase in vegetative mass, increase in yield, increase in the nutritional value of the grain has been demonstrated (Example 2-6, Tables 1-5).

In field tests, increase in the potato yield has also been observed with the use of Beijerinckia fluminensis strain Bf. 2806 (Example 7, Table 6).

In field tests of biomass of Beijerinckia fluminensis strain Bf 2806 on fruit trees, coniferous tree species, as well as flowers and ornamental plants, stimulation of growth of the vegetative mass has been noted (Examples 8-9).

In another variant, the Beijerinckia fluminensis strain Bf 2806 is used as a biological control agent in the prevention and/or treatment of fungal diseases of plants, particularly rhizoctonia (black scurf), one of the most harmful and common diseases of potatoes, and fusariosis, a common and dangerous infectious disease of cultivated and wild plants caused by fungi of the genus Fusarium.
Beijerinckia fluminensis strain Bf 2806, according to the invention, exerts a pronounced suppressive effect in open ground on the pathogens of winter wheat of fungal origin (Example 6, Table 5).

The use of Beijerinckia fluminensis strain Bf 2806, according to the invention, also has a significant effect on potato resistance to fungal diseases (Example 7, Table 6).
In one more variant, the invention relates to a method for stimulating plant growth and protection against diseases involving pre-sowing treatment of soil, seeds or crops with a suspension of Beijerinckia fluminensis strain Bf 2806, at a concentration of 1.0 to 5.0 billion cells/ml.

In presowing seed treatment, the preferred consumption rate of the working solution of Beijerinckia fluminensis strain Bf 2806 cells at a concentration of 1.0-5.0 billion cells/ml is 2.0-4.0 1 per ton of seeds of cereal crops (wheat, barley, oats, rye, rice, buckwheat); from 1.5 to 5 1 per ton of legumes and from 0.3 to 0.5 1 per ton of potato tubers.

In case of secondary tillage, the preferred consumption rate of the working solution Beijerinckia fluminensis strain Bf 2806 cells at a concentration of 1.0-5.0 billion cells/ml for all crops is 0.4 to 1.0 1/ha.

When fertilizing growing plants, the preferred consumption rate of the working solution is 0.4-1.0 1/ha for cereals, 0.4-1.0 1/ha for legumes, and from 0.4 to 1.0 1/ha for sugar beet, rice, maize, sunflower, buckwheat, rapeseed, mustard, and potatoes.

When treating fruit, berry, coniferous, deciduous, ornamental trees and shrubs, the presowing treatment of seeds is preferably carried out at the rate of 20-30 ml of Beijerinckia fluminensis strain Bf 2806 cells at a concentration of 1.0-5,0 billion cells/ml per 200 ml of water. The consumption rate of the working solution is 200 ml per 100 g seeds. When fertilizing young plants and seedlings, it is preferable to irrigate the roots with a solution of 30-50 ml of Beijerinckia fluminensis strain Bf 2806 cells at a concentration of 1.0-5.0 billion cells/ml per 5-10 1 of water.

The invention is illustrated by the following examples presented to confirm, but not to limit the scope of the claims.

### EXAMPLES

### Example 1. Culturing of Beijerinckia fluminensis strain Bf. 2806 (VKPM number B-12258)

A sterile liquid nutrient medium with the following composition, mass %: KH₂ PO₄ - 0.02-0.06; NaCl - 0.01-0.05; Fe₂(SO₄)₃ - 0.0001-0.0007; CaCO₃ - 0.1-0.8; molasses - 1.0-10.0; pH 6.0-7.5 is seeded in an inoculator with a cell culture of Beijerinckia fluminensis Bf 2806 (VKPM B-12258) at a rate of 10 ± 3 % of the volume of the culture medium. It is grown for 35 ± 5 hours at a temperature of 30 ± 5 °C, with continuous stirring and aeration at a rate of 1 volume of air / 1 volume of nutrient medium per minute.

The obtained seed culture is inoculated into a fermenter with a pre-sterilized liquid nutrient medium with a composition, mass %:
KH₂PO₄ - 0.04-0.1; NaCl - 0.01-0.05; MgSO₄ - 0.01-0.05; FeSO₄ - 0.0001-0.0007; MnSO₄ - 0.0001-0.0007; CaCO₃ - 0.1-0.8; molasses - 1.0-10.0; Corn extract - 0.25-0.5; pH 6.0-7.7 at the rate of 5-10 % of the volume of the nutrient medium. The culturing is conducted at a temperature of 28 ± 5 °C, with continuous stirring and aeration at a rate of 1 volume of air / 1 volume of nutrient medium per minute. Duration of culturing 50 ± 10 hours.

A liquid culture with a titer of 5-30 billion cells/ml and an IAA content (β-indolylacetic acid, a phytohormone of the auxin type) from 0.001 to 0.0001 µg/ml is obtained.

When carrying out the fermentation, the following should be added to the culture medium (mass %): (NH₄)₂MoO₇ - 0.0001-0.0008; H₃BO₃ - 0.0001-0.0007.

The finished fermentation product, which is a suspension of active bacterial cells of Beijerinckia fluminensis strain Bf 2806 (VKPM number B-12258) with a titer 1.0-5.0 billion cells/ml, is used as a fertilizer and a means for the prevention and/or treatment of agricultural and ornamental plants against fungal diseases.

The fertilizer is a product with the number of viable bacterial cells of Beijerinckia fluminensis strain Bf 2806 of at least 1.0-5.0 billion cells/ml, with a mass fraction of water not more than 97 %.

The finished product remains active for 12 months at a temperature of 0 to + 30 °C.

### Example 2. Stimulation of vegetative mass growth and increase in the yield of winter wheat

In 2014, field tests of fertilizers based on the Beijerinckia fluminensis strain were conducted in Krasnodar Region (Novopokrovsky District, Stanitsa Novopokrovskaya, Otkormochny-Ametist LLC). Biomass of Beijerinckia fluminensis strain Bf 2806 (VKPM B-12258) with a titer of 5-30 billion cells/ml was obtained under the conditions described in Example 1.

A single presowing treatment of winter wheat seeds of the Gratsia variety was carried out at a consumption rate of the bacterial product of 2.0 1 per 1 ton of seeds Spraying of crops during growth 0.5 1/ha. At the end of the growing season, the harvest was reaped and the total yield in dt/ha was estimated.

The test results are presented in Table 1.

**Table 1.**

| Criterion (earing phase) | Control | Test |
|---|---|---|
| Tillering capacity, qty | 3.2 | 4.7 |
| Yield, dt/ha | 62.59 | 67.79 |

The data in Table 1 demonstrate that the sowing of seeds treated with the fertilizer on the basis of Beijerinckia fluminensis strain Bf 2806 resulted in a significant increase in the vegetative mass during the earing phase, while the yield increase in the experimental plot in relation to the sowing of untreated seeds in the control plot was 11.7 %.

### Example 3. Stimulation of vegetative mass growth and increase in the yield of winter wheat.

In 2014, field tests of fertilizers based on the Beijerinckia fluminensis strain Bf 2806 were conducted in Krasnodar Region (Novokubansky District, Stanitsa Sovetskaya, Novator LLC).

Biomass of Beijerinckia fluminensis strain Bf 2806 VKPM B-12258 with a concentration of 1.0-5.0 billion cells/ml was obtained under the conditions described in Example 1.

A single treatment of winter wheat seeds of the Etnos variety was carried out during growth at the tillering stage, at a consumption rate of the bacterial product of 0.75 1 per 1 ha.

The test results are presented in Table 2.

**Table 2**

| 1. Criterion (ripening stage) | Control | Test |
|---|---|---|
| Ear length, cm | 12.7 | 13.5 |
| Number of ears | 22.7 | 25.0 |
| Flag leaf length, cm | 24.6 | 28.0 |
| Yield, dt/ha | 65.6 | 68.2 |

The data presented in Table 2 demonstrate that the sowing of seeds treated with the fertilizer on the basis of the Beijerinckia fluminensis strain Bf 2806 resulted in a significant increase in the vegetative mass during the ripening stage, while the yield increase in the experimental plot compared to the control plot was +12.8 %.

### Example 4. Stimulation of vegetative mass growth, increase in chlorophyll content and increase in the yield of winter wheat

In 2014, field tests of fertilizers based on the strain of Beijerinckia fluminensis were conducted in Rostov Region (Belokalitvinsky District, Ilyinka, Berezka LLC) with a presowing treatment of winter wheat seeds of the Gubernator Dona variety, at a consumption rate of the bacterial product of 2.5 1 per 1 t of seeds. Biomass of Beijerinckia fluminensis strain Bf 2806 VKPM B-12258 with a concentration of 1.0-5.0 billion cells/ml was obtained under the conditions described in Example 1.

The test results are presented in Table 3.

**Table 3.**

| 1. Criterion (earing phase) | Control | Test |
|---|---|---|
| Ear length, cm | 8.7 | 9.1 |
| Number of ears | 18.3 | 18.7 |
| Flag leaf length, cm | 15.7 | 19.2 |
| Chlorophyll content in leaves, mg/dm | 3.46 | 3.88 |

| 2. Criterion | Control | Test |
|---|---|---|
| Yield, (dt/ha) | 15.96 | 24.93 |

The data presented in Table 3 demonstrate that presowing treatment of seeds with the fertilizer based on Beijerinckia fluminensis strain Bf 2806 leads to a significant increase in vegetative mass during the earing phase, an increase in yield (the increase in yield at the experimental plot was + 56.7 % compared to the control plot) and an increase in the chlorophyll content in leaves measured using a chlorophyll content N-tester.

### Example 5. Stimulation of vegetative mass growth, increase in chlorophyll content and increase in the yield of winter wheat

In 2014, field tests of fertilizers based on the Beijerinckia fluminensis strain Bf 2806 were conducted in Stavropol Region (Trunovsky District, Donskoe Village, Trunovskoye OJSC) using a single treatment of winter wheat crops (tillering phase) of the Lebed variety at a consumption rate of the bacterial product of 0.97 1 per 1 hectare. Biomass of Beijerinckia fluminensis strain Bf 2806 (VKPM B-12258) at a concentration of 1.0-5.0 billion cells/cu. cm was obtained under the conditions described in Example 1.

The test results are presented in Table 4.

**Table 4**

| 1. Criterion (earing phase) | Control | Test |
|---|---|---|
| Ear length, cm | 9.7 | 10.1 |
| Number of ears | 19.1 | 20.3 |
| Flag leaf length, cm | 20.0 | 23.7 |
| Chlorophyll content in leaves, mg/dm | 3.06 | 3.6 |
| Yield, dt/ha | 38 | 42.1 |

The data presented in Table 4 demonstrate that a treatment of seeds with the fertilizer based on Beijerinckia fluminensis strain Bf 2806 at the tillering stage leads to a significant increase in the vegetative mass during the earing phase, an increase in yield (the increase in yield at the experimental plot was + 10.8 % compared to the control plot) and an increase in the chlorophyll content in leaves.

### Example 6. Increase in the yield of the winter wheat and suppression of pathogenic microflora

Biomass of Beijerinckia fluminensis strain Bf 2806 VKPM B-12258 with a concentration of 1.0-5.0 billion cells/ml was obtained under the conditions described in Example 1.

In 2014, field tests of fertilizers based on Beijerinckia fluminensis strain Bf 2806 were conducted in Krasnodar Region (Gulkevichsky District, Soyuz Agro LLC) using a single treatment of soil at an early stage of the growing season of winter wheat (Tanya variety) at a consumption rate of the product of 0.4 1 per 1 hectare.

The test results are presented in Table 5.

**Table 5**

| Criterion | Control | Test |
|---|---|---|
| Yield, (dt/ha) | 41.13 | 52.04 |
| Gluten (content in a seed, %) | 21.6 | 22.3 |
| Protein (content in a seed, %) | 14.6 | 14.8 |
| Fusarium wilt (prevalence of plants with affected ears, %) | 2.3 | 1.6 |

The data presented in Table 5 demonstrate that a treatment of crops with the fertilizer based on Beijerinckia fluminensis strain Bf 2806 leads to an increase in yield (the increase in yield in the experimental plot was + 24.1 % compared to the control plot), an increase in nutritional value (increase in protein and gluten content) with a clearly expressed suppressive effect of the product on pathogens of fungal origin. In the conducted tests, the number of affected ears decreased with a disease prevalence rate of 2.3 %.

### Example 6. Increase in potato yield and suppression of pathogenic microflora

In 2009, in a field test on the territory of the All-Russian State Research Institute for Potato Farming (VNIIKKH) "Korenevo", Lyubertsy District of Moscow Region, potato tubers were treated during planting of an early-ripening potato variety (Krepysh) with a product based on Beijerinckia fluminensis strain Bf 2806 (VKPM number B-12258). The planting was carried out in a soddy-podzolic, sandy loam soil characterized by an acid reaction of the medium and high hydrolytic acidity (pHKCl = 4.47-4.63; Hh = 4.25-4.52 meq / 100 g soil); low total absorbed bases and degree of their saturation (S = 4.2-4.9 meq / 100 g soil; V = 48.5-53.6 %); a content of mobile phosphorus at the level optimal for potatoes (213-227 mg/kg soil) and an average content of exchangeable potassium (165-192 mg/kg soil); and a relatively high humus content (2.39-2.64 % humus).

The yield of potatoes with the application of the product based on the strain was 36.3 t/ha against 25 t/ha in the control (the increase compared to control was 45.2 %).

Thus, the treatment of potato tubers with the product based on Beijerinckia fluminensis strain Bf 2806 leads to increased yield.

The use of the bacterial fertilizer according to the invention had a significant suppressive effect on the spread and development of fungal diseases on potato tubers, the results of the effect of the product on potato resistance to fungal diseases are presented in Table 2.

**Table 6. Prevalence and development of fungal diseases on potato tubers**

| | Rhizoctonia | |
|---|---|---|
| | Prevalence % | Development % |
| Control | 26.7 | 9.7 |
| Test | 15.2 | 4.0 |

The data presented in the Table 6 demonstrate the effectiveness of Beijerinckia fluminensis strain Bf. 2806 in the reduction of the number of potato tubers affected by rhizoctonia, with a prevalence rate of 26.7 % and development rate of 9.7 %.

### Example 7. Stimulation of vegetative mass and root system growth in fruit trees

In 2012, an experiment was conducted to test a fertilizer on the basis of Beijerinckia fluminensis strain in Tula Region, on the territory of the Research and Production Center of Biotechnology "Phytogenetics". Biomass of Beijerinckia fluminensis strain Bf 2806 VKPM B-12258 with a concentration of 1.0-5.0 billion cells/ml was obtained under the conditions described in Example 1.

Plants of cherry plum of the Iyulskaya Rosa variety, grown in pots, were planted in open soil. A month after planting they were treated with the fertilizer. The treatment was performed 3 times every 2 weeks. The experiment produced the following results:
- The height of the treated plants increased by an average of 64 % in comparison with the control of the Iyulskaya Rosa cherry plum variety
- the leaf blade area in plants to which the product was applied was 50 % larger than in plants without any treatments;
- the volume of the root system increased by 73 % in the treated plants.

### Example 8. Stimulation of vegetative mass growth in flowers and ornamental plants

In 2013, on the territory of the Main Botanical Garden (Moscow) an experiment was conducted to test a fertilizer on the basis of Beijerinckia fluminensis strain. Biomass of Beijerinckia fluminensis strain Bf 2806 (VKPM number B-12258 ) with a titer of 5-30 billion cells/ml was obtained under the conditions described in Example 1.

The treatment with the product was carried out in the form of watering the soil in a concentration of 50 ml / 10 1 of water. The agrochemical preoperties of soils of the experimental plots: pH - 6.3 in KCl, humus - 7.73 %, NO3 - 7.9 mg / 100 g, P2O5 - 50.1 mg / 100 g, K2O - 23.5 mg / 100 g.

The following results were obtained after four-fold fertilization from May to September:
- in comparison with the control (the parameters of which are taken as 100 %), the increase in deciduous rhododendrons treated with the fertilizer was 186.7 %, the increase in evergreen rhododendrons was 150.0 %.
- significant branching and tillering was noted under the influence of fertilization with the product, both in evergreen and deciduous rhododendrons.

### Example 9. Stimulation of vegetative mass growth in coniferous tree species

In 2012, an experiment was conducted on the territory of the Shchelkovo Training and Experimental Forest Range (Moscow Pegion), to test a fertilizer based on Beijerinckia fluminensis strain Bf 2806. Biomass of Beijerinckia fluminensis strain Bf 2806 (VKPM B-12258) at a concentration of 1.0-5.0 billion cells/cu. cm was obtained under the conditions described in Example 1.

In the plot of experimental production crops, the root systems of pine seedlings were treated once with a fertilizer suspension during planting. The following results were obtained:
- survival of seedlings: in the experimental (fertilizer-treated) plot - 97-98 %, in the control - 86 %;
- the width of the crown of the treated plants exceeded the control along the row by 57 %, and across the row by 28 %;
- the following parameters of the seedlings were identified in the experimental plot (control is taken as 100 %): height of seedlings - 113 %, seasonal growth - 129 %, trunk diameter - 200 %.

The aforementioned examples confirm that Beijerinckia fluminensis strain Bf 2806 proposed according to the invention is a highly effective fertilizer that stimulates the growth of the vegetative mass and root system of plants and increases the yield of agricultural crops and ornamental plants, as well as an anti-phytopathogenic agent for the prevention and/or treatment of plant diseases.

## Claims

1. The strain of bacteria Beijerinckia fluminensis Bf. 2806, deposited in VKPM under the number B-12258, used as a fertilizer and biological control agent for the prevention and/or treatment of fungal disease of agricultural and ornamental plants.

2. Use of Beijerinckia fluminensis strain Bf. 2806 VKPM B-12258 as a fertilizer for agricultural and ornamental plants.

3. Use of Beijerinckia fluminensis strain Bf. 2806 VKPM B-12258 as a biological control agent for the prevention and/or treatment of fungal disease of agricultural and ornamental plants.

4. Use according to claim **2,** wherein the plant is selected from a group consisting of cereals, potatoes, vegetables, berries, legumes, flowers and ornamental plants, coniferous and deciduous trees and shrubs.

5. Use according to claim **3,** wherein the fungal disease is rhizoctonia and fusariosis.

6. A method of growth stimulation and plant protection against fungal diseases comprising presowing treatment of soil, seeds or crops with microorganism cells **characterized in that** a suspension of Beijerinckia fluminensis Bf 2806 strain in a concentration of at least 1.0-5.0 billion cells/ml used as a biological control agent.

7. The method according to claim **6, characterized in that** the consumption rate of the working solution of Beijerinckia fluminensis Bf 2806 bacteria strain cells per ton of cereal crops seeds is 2.0-4.0 1, per ton of legumes - from 1.5 to 5 liters, per ton of potato tubers - from 0.3 to 0.5 liters

## Patentansprüche

1. Bakterienstamm Beijerinckia fluminensis Bf. 2806, eingetragen im VKPM unter der Nummer B-12258, zur Verwendung als Düngmittel und biologisches Kontrollmittel zur Vorbeugung und / oder Behandlung von Pilzbefall bei landwirtschaftlichen Pflanzen und Zierpflanzen.

2. Verwendung des Bakterienstammes Beijerinckia fluminensis Bf. 2806 VKPM B-12258 als Düngmittel bei landwirtschaftlichen Pflanzen und Zierpflanzen.

3. Verwendung des Bakterienstammes Beijerinckia fluminensis Bf. 2806 VKPM B-12258 als biologisches Kontrollmittel zur Vorbeugung und / oder Behandlung von Pilzbefall bei landwirtschaftlichen Pflanzen und Zierpflanzen.

4. Verwendung nach Anspruch **2,** wobei die Pflanze aus einer Gruppe ausgewählt wurde, die aus Getreide, Kartoffeln, Gemüse, Beeren, Hülsenfrüchten, Blumen und Zierpflanzen, Nadel- und Laubbäumen und Sträuchern besteht.

5. Verwendung nach Anspruch **3,** wobei Pilzbefall Rhizoktonie und Fusariose ist.

6. Verfahren zur Stimulierung des Wachstums und des Schutzes von Pflanzen gegen Pilzbefall, das die Behandlung des Bodens, der Samen oder der Saat vor dem Pflanzen mit Mikroorganismenzellen umfasst, **dadurch gekennzeichnet, dass** die Lösung des Stammes Beijerinckia fluminensis Bf. 2806 bei einer Konzentration von mindestens 1,0 bis 5,0 Milliarden Zellen / ml als biologisches Kontrollmittel verwendet wurde.

7. Verfahren nach Anspruch **6, dadurch gekennzeichnet, dass** die Verbrauchsrate der Arbeitslösung der Zellen des Stammes Beijerinckia fluminensis Bf. 2806 pro Tonne Getreidesamen 2,0 bis 4,0 1, pro Tonne Hülsenfrüchte von 1,5 bis 5 Liter, pro Tonne Kartoffelknollen von 0,3 bis 0,5 Liter beträgt.

## Revendications

1. Souche bactérienne Beijerinckia fluminensis Bf. 2806, entré dans VKPM sous le numéro B-12258, à utiliser comme engrais et agent de contrôle biologique pour la prévention et / ou le traitement des maladies fongiques des plantes agricoles et ornementales.

2. Utilisation de la souche Beijerinckia fluminensis Bf. 2806 VKPM B-12258 comme engrais pour plantes agricoles et ornementales.

3. Utilisation de la souche Beijerinckia fluminensis Bf. 2806 VKPM B-12258 comme agent de contrôle biologique dans la prévention et / ou le traitement des maladies fongiques des plantes agricoles et ornementales.

4. Utilisation selon la revendication **2,** où la plante est choisie dans le groupe comprenant les céréales, les pommes de terre, les légumes, les baies, les légumineuses, les fleurs et les plantes ornementales, les arbres et arbustes conifères et feuillus.

5. Utilisation selon la revendication **3,** où la maladie fongique est la rhizoctonie et la fusariose.

6. Procédé de stimulation de la croissance et de protection des plantes contre les maladies fongiques comprenant le traitement du sol, des graines ou des cultures avant la plantation avec des cellules de micro-organismes, **caractérisé en ce que** la solution de la souche Beijerinckia fluminensis Bf. 2806 à une concentration d'au moins 1,0 à 5,0 milliards de cellules / ml est utilisée comme agent de contrôle biologique.

7. Procédé selon la revendication **6, caractérisé en ce que** le taux de consommation de solution de travail de cellules de la souche Beijerinckia fluminensis Bf. 2806 est par tonne de graines de céréales 2,0-4,0 1, par tonne de légumineuses de 1,5 à 5 litres, par tonne de tubercules de pomme de terre de 0,3 à 0,5 litres.
